# EUROPÄISCHE PATENTSCHRIFT

(11) **EP 1 242 347 B1**
(45) Veröffentlichungstag und Bekanntmachung des Hinweises auf die Patenterteilung: **13.10.2004**
(21) Anmeldenummer: 00983239.5
(22) Anmeldetag: 07.12.2000
(51) Int. Cl.: C07C 37/66, C07F 9/54, C08G 64/00, C07C 39/02

(54) **VERFAHREN ZUR HERSTELLUNG VON PHOSPHONIUMPHENOLATEN**
METHOD FOR PRODUCING PHOSPHONIUM PHENOLATES
PROCEDE DE PRODUCTION DE PHENOLATES DE PHOSPHONIUM

(30) Priorität: 20.12.1999 DE 19961520
(43) Veröffentlichungstag der Anmeldung: 25.09.2002
(73) Patentinhaber: Bayer MaterialScience AG, 51368 Leverkusen (DE)
(72) Erfinder: BUNZEL, Lothar, 47906 Kempen (DE); HUCKS, Uwe, 46519 Alpen (DE); KÖNIG, Annett, 51371 Leverkusen (DE); KRATSCHMER, Silke, 47829 Krefeld (DE)
(86) Internationale Anmeldenummer: PCT/EP2000/012325
(87) Internationale Veröffentlichungsnummer: WO 2001/046100

(56) Entgegenhaltungen:
- EP-A- 0 826 693
- WO-A-99/00395

## Beschreibung

Die Anmeldung betrifft ein Verfahren zur Herstellung von Phosphoniumphenolaten.

Die Herstellung von Phosphoniumphenolaten ist bereits in der DE-A-197 273 51 beschrieben. Die so hergestellten Phosphoniumphenolate enthalten jedoch Verunreinigungen, die für den weiteren Einsatz von Nachteil sind. So ist bekannt, dass Phosphoniumphenolate als Umesterungskatalysatoren für die Schmelzeumesterung eingesetzt werden können. Hierbei spielt jedoch die Reinheit eine entscheidende Rolle, da Verunreinigungen zu Verfärbungen, Aktivitätsschwankungen oder Nebenreaktionen im Umesterungsprozeß führen können. Weiterhin fällt bei diesem Verfahren eine erhebliche Menge an Lösungsmittel als Abfallprodukt an, was hohe Entsorgungskosten mit sich bringt. Darüberhinaus wird das Produkt bei der Entfernung von Restlösungsmittel thermisch belastet. Da Phosphoniumphenolate leicht Addukte mit Phenol eingehen, ist eine Temperaturbelastung aber so gering wie möglich zu halten.

Aufgabe der vorliegenden Anmeldung ist somit ein Verfahren zu finden, in dem die Temperaturbelastung sowie die anfallenden Lösungsmittelmengen möglichst gering bleiben, die Reinheit des Phosphoniumphenolats aber möglichst hoch ist.

Gegenstand der Anmeldung ist ein Verfahren zur Herstellung von Phosphoniumphenolaten durch Umsetzung von Phosphoniumhalogeniden und Phenolen in wäßrig-alkalischer Lösung, dadurch gekennzeichnet, dass man das hergestellte Phosphoniumphenolat durch Kristallisation aus dem Synthesegemisch abtrennt.

Die Umsetzung wird vorzugsweise bei Temperaturen von 0 bis 55°C, insbesondere 15 bis 50°C durchgeführt.

Die Umsetzung wird vorzugsweise bei Molverhältnissen von Phenol zu Phosphoniumhalogenid von 2:1 bis 10:1, vorzugsweise von 4.5:1 bis 6:1 und insbesondere 5:1 durchgeführt.

Die Umsetzung wird vorzugsweise bei pH-Werten von 9.5 bis 11, vorzugsweise von 9.5 bis 10.5 und insbesondere von 10 bis 10.5 durchgeführt.

Die Umsetzung wird gegebenenfalls in Gegenwart von Alkoholen in Mengen von 50 Gew.-% bis 200 Gew.-%, vorzugsweise von 66 Gew.-% bis 125 Gew.-%, bezogen auf Gewichtsmenge wäßrige Phase durchführt, wobei die Alkohole vorzugsweise eine Löslichkeit in reinem Wasser von maximal 15 Gew.-% haben.

Die derart hergestellten Phosphoniumphenolate enthalten nicht mehr als 0. Gew.-% Halogenid.

Für die Umsetzung werden insbesondere Phosphoniumhalogenide der Formel (I) 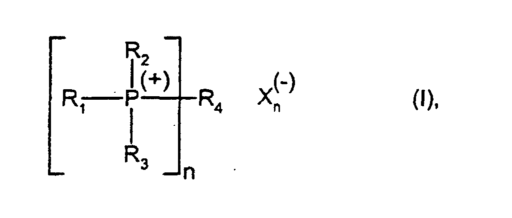 worin
- R₁ bis R₄: gleich oder verschieden, jeweils für einen C₁-C₁₂-Alkyl-, C₅-C₆-Cycloalkyl-, C₇-C₁₂-Aralkyl- oder C₆-C₁₄-Aryl-Rest stehen, und
- X⁽⁻⁾: für ein Halogenion, vorzugsweise F⁽⁻⁾, Cl⁽⁻⁾ oder Br⁽⁻⁾ steht, und
- n: für die Zahl 1 oder 2 steht, wobei für n = 2 R₄ für einen C₂-C₁₂-Alkylenrest steht.

Bevorzugt sind die Reste R₁ bis R₄, gleich oder verschieden, und stehen jeweils für einen C₆-C₁₄-Aryl-Rest oder die Reste R₁ bis R₃ stehen jeweils für einen C₆-C₁₄-Arylrest und R₄ für einen C₂-C₁₂-Alkylenrest.

Derartige Phosphoniumhalogenide und ihre Herstellung sind bekannt oder nach bekannten Methoden erhältlich (siehe beispielsweise "Houben-Weyl, Methoden der organischen Chemie" Band XII/1, Seiten 79 ff. und Worrall, J. Amer. Chem. Soc. 52 (1930), Seiten 293 ff.).

Diese Verbindungen (I) entstehen bei der Umsetzung von Trialkyl- oder Triarylphosphinen, beispielsweise von Triphenylphosphin mit Halogenarylen oder Halogenalkylen, z.B. Benzylbromid in Gegenwart von Metallsalzen (Friedel-Crafts-Alkylierung) oder in Gegenwart von Grignard-Verbindungen und Kobalt(II)-chlorid.

Für die Umsetzung bevorzugte Phenole sind Phenol oder substitutierte Phenole sowie Bisphenole.

Besonders bevorzugte Phenole sind die der Formel (II) 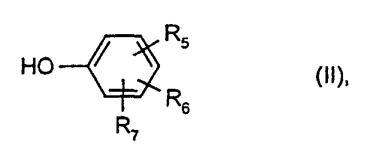 worin
- R₅ bis R₇: unabhängig voneinander für H, C₁-C₁₂-Alkyl, C₅-C₆-Cycloalkyl, C₇-C₁₂-Arylalkyl und C₆-C₁₄-Aryl stehen; vorzugsweise stehen R₅ bis R₇ für Wasserstoff.

Derartige Phenole sind literaturbekannt.

Bevorzugte werden Phosphoniumphenolate der Formel (III) hergestellt 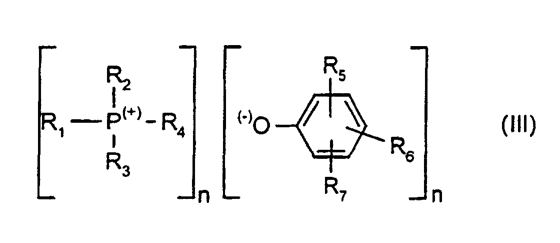 worin die Reste R₁ bis R₇ und n die oben angegebenen Bedeutungen haben.

Für die Herstellung der wäßrig-alkalischen Phase wird bevorzugt VE-Wasser bzw. destilliertes Wasser verwendet.

Der pH-Wert von 9.5 bis 11.0, vorzugsweise von 9.5 bis 10.5, besonders bevorzugt von 10.0 bis 10.5, wird vorzugsweise mit Hilfe einer Alkalilauge, vorzugsweise Natronlauge oder Kalilauge - unter Berücksichtigung der puffernden Wirkung von Phenol/Na-Phenolat - eingestellt.

Das erfindungsgemäße Verfahren kann kontinuierlich oder diskontinuierlich durchgeführt werden, wobei eine diskontinuierliche Arbeitsweise bevorzugt wird.

Nach einer bevorzugten Arbeitsweise werden Phenol, Phosphoniumhalogenid und Alkohol als Lösung vorgelegt und Wasser zugegeben. Gegebenenfalls unter Kühlung wird der pH-Wert unter Zugabe von Alkalilauge auf Werte von 9.5 bis 11.0, vorzugsweise von 9.5 bis 10.5, besonders bevorzugt von 10.0 bis 10.5 eingestellt. Dabei wird vorzugsweise unter intensiver Mischung der Reaktionskomponenten die Temperatur von 0 bis 55°C, vorzugsweise 15 bis 50°C gehalten. Die Reaktionsdauer sollte unter 2 Stunden, vorzugsweise unter 1 Stunde liegen.

Das erfindungsgemäß hergestellte Phosphoniumphenolat wird isoliert, vorzugsweise indem man unter Verwendung eines in Wasser-schwerlöslichen Alkohols (die Löslichkeit der Alkohole in Wasser ist literaturbekannt) die wäßrige Phase von der organischen Phase trennt und letztere mindestens 1 mal, vorzugsweise 3 mal mit VE-Wasser bzw. destilliertem Wasser extrahiert. Anschließend wird die Lösung auf 26 bis 0°C, vorzugszweise auf 23 bis 10°C abgekühlt. Das ausgefallene Phenolat wird anschließend abgesaugt und durch Auswaschen gereinigt. Gegebenenfalls wird das anfallende Produkt umkristallisiert und getrocknet.

Erfindungsgemäß geeignete Alkohole für die Reaktionslösung sind aliphatische der Formel CₙH₂ₙ₊₁-OH, worin n eine ganze Zahl von 4 bis 10 einschließlich ist, wie z. B. n-Butanol, Isobutanol, n-Pentanol, Methylbutanole, Neopentanol, Amylalkohole, verzweigte und unverzweigte Hexanole, Heptanole, Octanole, Nonanole oder Decanole.

Erfindungsgemäß geeignete Alkohole für die Reaktionslösung sind auch cycloaliphatische der Formel CₙH₂ₙ₋₁-OH, worin n eine ganze Zahl von 5 bis 10 einschließlich ist, wie z. B. Cyclopentanol, Methylcyclopentanole, Cyclopentanmethanol, Cyclopentylpropanole, Cyclohexanol, Cyclohexylethanole, Cyclohexylpropanole, Cyclohexylbutanole, Methyl-, Ethyl-, Propyl- und Butylhexanole, Cycloheptanole, Cyclooctanole.

Für das Auswaschen des Phenolates können neben VE-Wasser ebenfalls Alkohole eingesetzt werden. Hier können neben denen für die Reaktionslösung auch in Wasser lösliche Alkohole benutzt werden, wie z. B. Ethanol, n-Propanol oder Isopropanol. Bevorzugt werden hier Propanole, insbesondere Isopropanol.

Erfindungsgemäß können auch mehrwertige aliphatische oder cycloaliphatische Alkohole eingesetzt werden.

Bevorzugte aliphatische Alkohole sind Propanole, (Iso)Butanole, Pentanole und Hexanole, insbesondere Isobutanol und Isopropanol.

Bevorzugte cycloaliphatische Alkohole sind Cyclopentanol, Cycloheptanol und Cyclooctanol, besonders bevorzugt Cyclohexanol.

Das Gewichtsverhältnis von Wasser zu Alkohol liegt zwischen 2:1 und 1:2, vorzugsweise zwischen 1:1 und 1:2.

Die erfindungsgemäß einzusetzenden Alkohole werden zur besseren Aufarbeitung zugesetzt, da die Mischung Phenol/Alkohol eine geringere Dichte als die wäßrige Lösung hat und somit die organische über der wäßrigen Phase ist. Somit kann die wäßrige Phase unten abgelassen werden, die organische Phase, die das Phenolat enthält, kann dann im selben Trenngefäß mit VE-Wasser gewaschen werden und das Waschwasser wiederum unten abgelassen werden.

Setzt man den Alkohol nicht zu, dann ist nur die salzhaltige wäßrige Lösung schwerer als die organische Phase und kann unten abgelassen werden. Bei der weiteren Wäsche mit VE-Wasser kommt es zur Phasenumkehr, die organische Phase ist schwerer und daher unter der wäßrigen Phase. Diese Aufarbeitung erweist sich dahingehend als aufwendiger, dass ein zweites Aufarbeitungsgefäß notwendig ist.

Erfindungsgemäß hergestellte quartäre Phosphoniumphenolate sind insbesondere die Verbindungen der Formeln 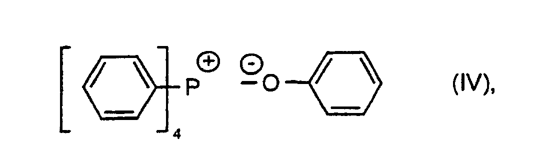 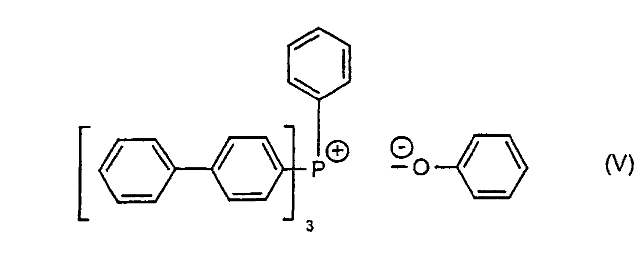 und

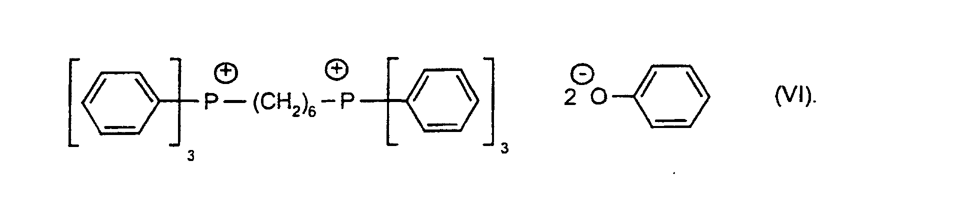

Mit dem erfindungsgemäßen Verfahren gelingt es, Phosphoniumphenolate in hohen Ausbeuten und hoher Reinheit herzustellen.

Die so hergestellten Phosphoniumphenolate eignen sich besonders als Katalysatoren zur Veresterung und zur Umesterung, insbesondere zur Herstellung von Polycarbonaten nach dem Schmelzumesterungsverfahren (siehe US-3 442 854).

Das Schmelzumesterungsverfahren geht bekanntlich beispielsweise aus von aromatischen Diphenolen, Kohlensäurediarylestern und gegebenenfalls Verzweigern und/oder Monophenolen.

Die erfindungsgemäß erhältlichen Phosphoniumphenolate werden hierbei als Katalysatoren in Mengen von 10⁻¹ mol bis 10⁻⁸ mol, vorzugsweise in Mengen von 10⁻³ mol bis 10⁻⁷ mol, pro mol Diphenol eingesetzt.

Weitere Einzelheiten des Schmelzumesterungsverfahrens sind in der Literatur beschrieben (siehe beispielsweise Hermann Schnell, Chemistry and Physics of Polycarbonates, Polymer Reviews, Vol. 9, 1964, Seiten 44 bis 51, DE-A-1 031 512, US-3 022 272, US-5 340 905 und US-5 399 659).

Die mit den erfindungsgemäß erhältlichen Phosphoniumphenolaten hergestellten thermoplastischen Polycarbonate sind lösungsmittelfrei, mit heller Eigenfarbe ausgestattet und weitgehend frei von unerwünschten Fehlstellen im Polycarbonat.

Der technische Einsatz dieser so hergestellten Polycarbonate in Form der verschiedensten Formteile ist überall dort möglich, wo bislang bereits thermoplastische Polycarbonate eingesetzt wurden, etwa in der Elektrotechnik, als Lampenabdeckungen, als Sicherheitsscheiben oder als optische Datenspeicher, wie CD-Material.

### Beispiele

### Vergleichsbeispiel 1

In einem 2-1-Rundkolben mit Rührer, Thermometer und Tropftrichter werden 376 g (4,0 mol) Phenol, 800 ml VE-Wasser, 335,44 g (0,8 mol) Tetraphenylphosphoniumbromid und 640 g Isobutanol vorgelegt und bei 20°C bis 25°C gerührt. Innerhalb von ca. 5 Minuten werden 79 g (0,97 mol) 49%ige Natronlauge zugetropft, der pH-Wert wird mit einer Glaselektrode kontrolliert, er muß in einem Bereich von 9,5 bis 11,0 liegen. Anschließend wird 0,5 h bei 45°C gerührt. Nach der Phasentrennung wird die untere wäßrige Phase abgelassen und die organische Phase dreimal mit 3E-Wasser gewaschen, das Waschwasser als schwerere Phase kann jeweils unten abgelassen werden. Anschließend wird das Isobutanol bei 50°C im Wasserstrahlvakuum abdestilliert.

Der kristalline Rückstand wird bei 100°C im Vakuum getrocknet. Die Ausbeute bestimmt nach der P-NMR Methode beträgt 98,2 % der theoretischen Ausbeute. Die Ergebnisse sind in Tabelle 1 aufgeführt.

### Beispiel 1

In einem 2-1-Rundkolben mit Rührer, Thermometer und Tropftrichter werden 376 g (4,0 mol) Phenol, 800 ml 3E-Wasser, 335,44 g (0,8 mol) Tetraphenylphosphoniumbromid und 640 g Isobutanol vorgelegt und bei 20°C bis 25°C gerührt. Innerhalb von ca. 5 Minuten werden 79 g (0,97 mol) 49%ige Natronlauge zugetropft, der pH-Wert wird auf einen Bereich von 9,5 bis 11,0 einghestellt. Anschließend wird 0,5 h bei 45°C gerührt. Nach der Phasentrennung wird die untere wäßrige Phase abgelassen und die organische Phase dreimal mit VE-Wasser gewaschen, das Waschwasser als schwerere Phase wird jeweils unten abgelassen. Anschließend wird die organische Phase unter Rühren auf Raumtemperatur abgekühlt. Dabei kristallisiert das Produkt aus. Nach mindestens 4 h Kristallisationszeit wird das Produkt abgesaugt. Das Filtrat wird nach NMR-Analyse auf den Gehalt an Phenol, Isobutanol und Tetraphenylphosphoniumphenolat wieder der Reaktion zugeführt. Der kristalline Rückstand wird mit 2-Propanol nachgewaschen, dann bei 100 °C im Wasserstrahlvakuum getrocknet.

Die Ergebnisse sind in Tabelle 1 aufgeführt.

**Tabelle 1**

| | **Vergleichsbsp. 1** | **Bsp. 1** |
|---|---|---|
| Na [ppm] | 0,7 | < 0,5 |
| Br [ppm] | 0,7 | 0,01 |
| Farbe | hellgrau | farblos |

### Verwendungsbeispiele

B1) In einem 500 ml Dreihalskolben mit Rührer, Innenthermometer und Vigreuxkolonne (30 cm, verspiegelt) mit Brücke werden 114,15 g (0,500 mol) Bisphenol A und 113,54 (0,530 mol) Diphenylcarbonat eingewogen. Die Apparatur wird durch Anlegen von Vakuum und Spülen mit Stickstoff (3 mal) vom Luftsauerstoff befreit und das Gemisch auf 150°C augeheizt. Jetzt werden 0,0173 g (4 x 10⁻³ mol-%) Tetraphenylphosphoniumphenolat (TPP-P) hergestellt gemäß Beispiel 1, bezogen auf Bisphenol A, als 3%ige phenolische Lösung zugegeben und das entstehende Phenol bei 100 mbar abdestilliert. Gleichzeitig wird die Temperatur bis auf 250°C gesteigert. Nun wird das Vakuum stufenweise bis auf 1 mbar verbessert und die Temperatur auf 260°C erhöht. Anschließend wird die Temperatur auf 280°C erhöht und bei 0,1 mbar 1,5 Stunden gerührt. Man erhält ein farbhelles lösungsmittelfreies Polycarbonat *mit einer relativen Lösungsviskosität von 1,250 (Dichlormethan, 25°C, 5 g*/*l).*
   Der Gehalt an Verzweiger der Formel (VII) im hergestellten Polycarbonat beträgt 25 ppm. Der phenolische OH-Wert des Polycarbonats beträgt 70 ppm. 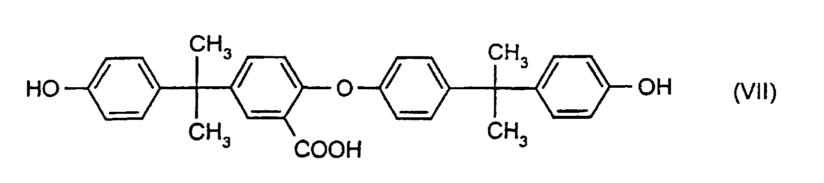
B2) Wie Beispiel B1), nur wird die Temperatur von 260°C auf 300°C erhöht und bei 0,1 mbar 1,5 Stunden gerührt. Man erhält ein farbhelles, lösungsmittelfreies Polycarbonat mit einer relativen Lösungsviskosität von 1,300 (Dichlormethan, 25°C, 5 g/l). Der Gehalt an Verzweiger der Formel (VII) im hergestellten Polycarbonat beträgt 18 ppm. Der phenolische OH-Wert des Polycarbonats beträgt 55 ppm.

## Patentansprüche

1. Verfahren zur Herstellung von Phosphoniumphenolaten durch Umsetzung von Phosphoniumhalogeniden und Phenolen in wäßrig-alkalischer Lösung, **dadurch gekennzeichnet, dass** man das hergestellte Phosphoniumphenolat durch Kristallisation aus dem Synthesegemisch abtrennt.

2. Verfahren gemäß Anspruch 1, **dadurch gekennzeichnet, dass** die Umsetzung bei Temperaturen von 0 bis 55°C durchgeführt wird.

3. Verfahren gemäß mindestens einem der Anspruch 1 oder 2, **dadurch gekennzeichnet, dass** die Umsetzung bei Molverhältnissen von Phenol zu Phosphoniumhalogenid von 2:1 bis 10:1, durchgeführt wird.

4. Verfahren gemäß mindestens einem der Anspruch 1 bis 3, **dadurch gekennzeichnet, dass** die Umsetzung bei pH-Werten von 9.5 bis 11 durchgeführt wird.

5. Verfahren gemäß mindestens einem der Ansprüchen 1 bis 4, **dadurch gekennzeichnet, dass** man in die Umsetzung in Gegenwart von Alkoholen in Mengen von 66 Gew.-% bis 125 Gew.-% durchführt.

6. Verfahren gemäß mindestens einem der Ansprüchen 1 bis 5, **dadurch gekennzeichnet, dass** die Kristallisation aus alkoholischer Lösung erfolgt.

7. Verfahren nach Anspruch 6, **dadurch gekennzeichnet, dass** der verwendete Alkohol Isobutanol ist.

8. Verfahren gemäß mindestens einem der Ansprüchen 1 bis 7, **dadurch gekennzeichnet, dass** die verwendete organische Phase kontinuierlich im Kreis geführt wird.

## Claims

1. Process for the production of phosphonium phenolates by reacting phosphonium halides and phenols in an aqueous alkaline solution, **characterised in that** the phosphonium phenolate produced is separated from the synthesis mixture by crystallisation.

2. Process according to claim 1, **characterised in that** the reaction is performed at temperatures of 0 to 55°C.

3. Process according to at least one of claims 1 or 2, **characterised in that** the reaction is performed at molar ratios of phenol to phosphonium halide of 2:1 to 10:1.

4. Process according to at least one of claims 1 to 3, **characterised in that** the reaction is performed at pH values of 9.5 to 11.

5. Process according to at least one of claims 1 to 4, **characterised in that** the reaction is performed in the presence of alcohols in quantities of 66 wt.% to 125 wt.%.

6. Process according to at least one of claims 1 to 5, **characterised in that** crystallisation proceeds from an alcoholic solution.

7. Process according to claim 6, **characterised in that** the alcohol used is isobutanol.

8. Process according to at least one of claims 1 to 7, **characterised in that** the organic phase used is continuously recirculated.

## Revendications

1. Procédé de préparation de phénolates de phosphonium par mise en réaction d'halogénures de phosphonium et de phénols dans une solution alcaline aqueuse, **caractérisé en ce que** l'on sépare le phénolate de phosphonium produit par cristallisation à partir du mélange de synthèse.

2. Procédé selon la revendication 1, **caractérisé en ce que** la mise en réaction est réalisée à des températures de 0 à 55°C.

3. Procédé selon au moins une des revendications 1 ou 2, **caractérisé en ce que** la mise en réaction est effectuée à des rapports molaires du phénol à l'halogénure de phosphonium de 2:1 à 10:1.

4. Procédé selon au moins une des revendications 1 à 3, **caractérisé en ce que** la mise en réaction est effectuée à des pH de 9,5 à 11.

5. Procédé selon au moins une des revendications 1 à 4, **caractérisé en ce que** la mise en réaction est effectuée en présence d'alcools dans des quantités de 66% en poids à 125% en poids.

6. Procédé selon au moins une des revendications 1 à 5, **caractérisé en ce que** la cristallisation intervient à partir de la solution alcoolique.

7. Procédé selon la revendication 6, **caractérisé en ce que** l'alcool utilisé est l'isobutanol.

8. Procédé selon au moins l'une des revendications 1 à 7, **caractérisé en ce que** la phase organique mise en oeuvre est introduite dans le cycle en continu.
